# EUROPEAN PATENT APPLICATION

(11) **EP 1 658 837 A1**
(43) Date of publication of application: **24.05.2006**
(21) Application number: 05023934.2
(22) Date of filing: 03.11.2005
(51) Int. Cl.: A61K 8/34, A61K 8/73, A61K 8/81, A61Q 1/00, A61Q 1/06, A61Q 17/00, A61Q 19/00

(54) **Moisturizing compositions**

(30) Priority: 04.11.2004 US 624540 P
(71) Applicant: L'OREAL, 75008 Paris (FR)
(72) Inventor: Yu, Wei Hong, Edison, New Jersey 08820 (US)
(74) Representative: Le Coupanec, Pascale A.M.P.

(57) **Abstract**

The present invention relates to compositions useful for moisturizing keratinous materials which contain poly(2-hydroxyethyl methacrylate) and at least one polyol.

## Description

The present invention relates to compositions useful for moisturizing the skin and/or hair which contain a poly(2-hydroxyethyl methacrylate) polyHEMA and at least one polyol.

Moisturizing compositions are known and widely used in the art. Their applicability ranges from providing moisture to lips, skin, hair, and/or nails as a treatment when they are dry or as a preventative or for the maintenance of the lips, skin, hair and/or nails as a part of an individual's routine skin and/or hair care regimen. For example, lip care compositions are commonly used not only to provide a make-up effect, e.g., color, but also to protect, soothe and/or moisturize. Polyols, such as for example, glycerin are known humectants which provide a moisture benefit to the lips, skin, hair, and/or nails when applied.

U.S. patent no. 5,855,876 describes moisturizing lipstick compositions that are long lasting and essentially free of water with lipophilic materials, such as hydrocarbon oils, fatty acid esters and fatty alcohols, moisturizers, colorants, and coupling agents, which are agents that have a function to interact with the hydrophobic lipophilic material and a function to interact with a hydrophilic moisturizer.

U.S. patent no. 5,498,407 describes the biocompatibility of poly(2-hydroxyethylmethacrylate) and their use in cosmetic compositions.

U.S. patent no. 6,045,783 describes cosmetic compositions that contain sodium polyacrylate polymers, that when combined with water or moisture swells up to several times beyond its original size. This cosmetic when applied to, e.g., the lips, makes the lips appear fuller.

Notwithstanding the compositions that were known previously, there remains a need for moisturizing compositions with at least one polyol that provide an exceptional moisturizing effect over longer periods of times with little or no adverse effects.

The inventors have discovered that exceptional, long-lasting moisturizing benefits can be achieved on keratinous material by applying a composition combining at least one polyol and poly(2-hydroxyethyl methacrylate).

Thus, the present invention provides a composition comprising a moisturizing effective amount of at least one polyol and poly(2-hydroxyethyl methacrylate).

The invention also provides methods of providing a cosmetic moisturizing benefit to an individual with the composition.

The invention also provides methods of making the composition.

The invention, in one embodiment, relates to cosmetic, dermatological, and pharmaceutical products containing a composition comprising moisturizing effective amounts of poly(2-hydroxyethyl methacrylate) and at least one polyol. The combination of a composition at least one polyol and poly(2-hydroxyethyl methacrylate) when applied to the skin, lips, hair and/or nails is that the composition imparts a superior moisturizing benefit, with little or no drying, no feathering of the composition, and a longer hydration effect. The composition is also believed to form a water and/or oil resistant film after the absorption of water.

The at least one polyol is incorporated into the composition such that when in combination with the poly(2-hydroxyethyl methacrylate) it provides a moisturizing benefit to the skin, lips and/or keratinous material of the user greater than the at least one polyol alone. The at least one polyol in combination with the poly(2-hydroxyethyl methacrylate) is provided in a moisturizing effective amount, which means an increase in perceived moisture on the lips, skin, hair or other keratinous materials after the composition of the invention is applied thereto. The concentrations, however, should not be such that water is pulled away from the keratinous material to which the composition is applied.

In another embodiment, moisture can be determined empirically using devices designed to measure moisture content, such as the SkinChip® sensor (L'Oréal and STMicroelectronics) or Novameter™ (NovaTechnology, Gloucester, MA). Using such a moisture testing device, the keratinous material onto which the composition described herein has been applied can benefit from at least a 5% increase in moisture relative to the moisture prior to application, which includes at least 10%, 15%, 20%, 25%, 30%, 40%, 50%, all subranges and values there between, or more.

In one embodiment, the at least one polyol may be present in amounts from 0.5 to 75% by weight of the composition, inclusive of 1, 5, 10, 15, 20, 25, 30, 35, 40, 45, 50, 55, 60, 65, and 70% by weight of the composition, and all ranges and sub ranges there between. In a preferred embodiment, the at least one polyol is present in an amount from about 1 to about 5%, including 2%, 3%, 4% and all ranges and subranges there between.

The at least one polyol used in the present invention is a polyol that is used as humectants and/or moisturizers in skin care compositions. Examples include hydrophilic and comprise polyhydric alcohols, ethoxylated and propoxylated polyols, polysaccharides, and mixtures thereof. Preferred polyols/moisturizers are selected from the group consisting of glycerin, panthenol, hexylene glycol, propylene glycol, butylene glycol, alkanediols, polyethylene glycol, polypropylene glycol, sorbitol, and mixtures thereof. Most preferred is glycerin.

Poly(2-hydroxyethyl methacrylate) is a skin-friendly, biocompatible polymer with a high moisture vapor transmission rate of about 2156 g/m²/day The poly(2-hydroxyethyl methacrylate) may be present in the inventive compositions such that when combined with at least on polyol provides a moisturizing effect. For example, the poly(2-hydroxyethyl methacrylate) may be in an amount ranging from about 0.1 to about 50% by weight, relative to the total weight of the composition, or from about 0.5 to about 40% by weight, or from about 1 to about 30% by weight. In a preferred embodiment, the poly(2-hydroxyethylmethacrylate) is present in the composition in an amount from about 1 to about 5%, including 2%, 3%, 4% and all values and subranges there between. In one embodiment, the poly(2-hydroxyethyl methacrylate) added to the composition is a solid powder.

The invention applies not only to make-up products for at least one keratinous material such as lip compositions, lip pencils, foundations including foundations which may be cast in the form of a stick or a dish, concealer products, temporary tattoo products, eyeliners, mascara bars but also to body hygiene products such as deodorant sticks, and to care products and products for treating at least one keratinous material such as sunscreen and after-sun products which may be in stick form. The present invention may be in the form of mascara product including mascara bars, an eyeliner product, a foundation product, a lipstick product, a liquid lip care product, a blush for cheeks or eyelids, a deodorant product, a make-up product for the body, a make-up-removing product, an eyeshadow product, a face powder product, a concealer product, a treating shampoo product, a hair conditioning product, a sun screen, colorant for the skin or hair, or skin care formula such as, for example, anti-pimple or shaving cut formulas. As defined herein, a deodorant product is a body hygiene product and does not relate to care, make-up, or treatment of keratin materials, including keratin fibers, skin, or lips.

For example, the composition of the present invention may be in a form chosen from a paste, a solid, a gel, and a cream. It may be an emulsion, such as an oil-in-water or water-in-oil emulsion, a multiple emulsion, such as an oil-in-water-in-oil emulsion or a water-in-oil-in-water emulsion, or a solid, rigid or supple gel, including anhydrous gels. In one embodiment, the composition of the invention is anhydrous. The composition of the invention may, for example, comprise an external or continuous fatty phase. In another embodiment, the composition of the invention is transparent or clear, including for example, a composition without pigments. The composition can also be in a form chosen from a translucent anhydrous gel and a transparent anhydrous gel. The composition can also be a molded composition or cast as a stick or a dish. The composition in one embodiment is a solid such as a molded stick or a poured stick, which can be anhydrous.
According to the instant invention, anhydrous means that the composition contains less than 5%, in particular less than 3% and more particularly less than 1% by weight of water with respect to its total weight. In particular, it may be free of water.

The compositions may also contain one or more film-forming polymers.

The film-forming polymer of the compositions may be chosen from any silicone resin that has film forming properties and any other organic material with film forming properties.

The film forming polymer of the compositions according to the present invention may also be a non silicone film former. This non silicone film former may be chosen from, for example, polyethylene; vinylpyrrolidone/vinyl acetate (PVP/VA) copolymers such as the Luviskol® VA grades (all ranges) from BASF® Corporation and the PVP/VA series from ISP; acrylic fluorinated emulsion film formers including Foraperle® film formers such as Foraperle® 303 D from Elf Atochem (although Foraperle® may not be appropriate for some cosmetic formulations); GANEX® copolymers such as butylated PVP, PVP/Hexadecene copolymer, PVP/Eicosene copolymer or tricontanyl; Poly(vinylpyrrolidone/diethylaminoethyl methacrylate) or PVP/Dimethylaminoethylmethacrylate copolymers such as Copolymer 845; Resin ACO-5014 (Imidized lB/MA copolymer); other PVP based polymers and copolymers; alkyl cycloalkylacrylate copolymers *(See* WO 98/42298, the disclosure of which is hereby incorporated by reference); Mexomere® film formers and other allyl stearate/vinyl acetate copolymers (allyl stearate/VA copolymers); polyolprepolymers such as PPG-12/SMDI copolymer, polyolprepolymers such as PPG-1 2/SM D1 copolymer, Poly(oxy-1,2-ethanediyl), α-hydro-ω-hydroxy-polymer with 1,1'-methylene-bis-(4- isocyanatocyclohexane) available from Barnet; Avalure™ AC Polymers (Acrylates Copolymer) and Avalure™ UR polymers (Polyurethane Dispersions), available from BFGoodrich.

The film former which also may be used within the framework of the invention includes film formers having any film former chemistry known in the art such as: PVP, acrylates, and urethanes; synthetic polymers of the polycondensate type or free-radical type, or ionic type, polymers of natural origin and mixtures thereof or any other film former known within the practice of the cosmetic and pharmaceutical arts which one skilled in the art may determine to be compatible. Film formers that may be used are also disclosed in the *International Cosmetic Ingredient Dictionary and Handbook Vol.* 2 (7^{th} ed. 1999), more particularly the emollients disclosed on pages 1636-1638. The disclosure of the *International Cosmetic Ingredient Dictionary and Handbook Vol. 2,* pages 1636-1638, is hereby incorporated by reference.

An appropriate concentration of the film former may be determined by one of skill in the art and can vary considerably based on the application. For example, for cosmetic compositions, the film former may be used in an amount from 0.1 % to 20% such as, for example, from 1% to 10% by weight, relative to the total weight of the composition.

Depending on the intended application, such as a stick, hardness of the composition may also be considered. The hardness of a composition may, for example, be expressed in grams (g). The composition of the present invention may, for example, have a hardness ranging from 20 g to 2000 g, such as from 20 g to 900 g, and further such as from 20 g to 600 g.

This hardness is measured in one of two ways. A first test for hardness is according to a method of penetrating a probe into said composition and in particular using a texture analyzer (for example TA-XT2 from Rhéo) equipped with an ebonite cylinder of height 25 mm and diameter 8 mm. The hardness measurement is carried out at 20°C at the center of 5 samples of said composition. The cylinder is introduced into each sample of composition at a pre-speed of 2 mm/s and then at a speed of 0.5 mm/s and finally at a post-speed of 2 mm/s, the total displacement being 1 mm. The recorded hardness value is that of the maximum peak observed. The measurement error is ± 50g.

The second test for hardness is the "cheese wire" method, which involves cutting an 8.1 mm tube of composition and measuring its hardness at 20°C using a DFGHS 2 tensile testing machine from Indelco-Chatillon Co. at a speed of 100 mm/minute. The hardness value from this method is expressed in grams as the shear force required to cut a stick under the above conditions. According to this method, the hardness of compositions according to the present invention which may be in stick form may, for example, range from 30 g to 300 g, such as from 30 g to 250 g, and further such as from 30 g to 200 g.

The hardness of the composition of the present invention may be such that the compositions are self-supporting and can easily disintegrate to form a satisfactory deposit on a keratinous material. In addition, this hardness may impart good impact strength to the inventive compositions which may be molded or cast, for example, in stick or dish form.

The skilled artisan may choose to evaluate a composition using at least one of the tests for hardness outlined above based on the application envisaged and the hardness desired. If one obtains an acceptable hardness value, in view of the intended application, from at least one of these hardness tests, the composition falls within the scope of the invention.

According to the present invention, the compositions in stick form may also possess the properties of deformable, flexible elastic solids and may also have noteworthy elastic softness upon application to a keratinous material.

The compositions of the present invention may also contain one or more volatile oils or solvents. For the purposes of the invention, the expression "volatile solvent or oil" means any non-aqueous medium capable of evaporating on contact with the skin or the lips in less than one hour at room temperature and atmospheric pressure. The volatile solvent(s) of the invention is(are) organic solvents, such as volatile cosmetic oils that are liquid at room temperature, having a non-zero vapor pressure, at room temperature and atmospheric pressure, ranging in particular from 10⁻² to 300 mmHg and, for example, greater than 0.3 mmHg. The expression "non-volatile oil" means an oil which remains on the skin or the lips at room temperature and atmospheric pressure for at least several hours, such as those having a vapor pressure of less than 10⁻² mmHg.

According to the invention, these volatile solvents may facilitate the staying power or long wearing properties of the composition on the skin, the lips or superficial body growths. The solvents can be chosen from hydrocarbon-based solvents, silicone solvents optionally comprising alkyl or alkoxy groups that are pendant or at the end of a silicone chain, and a mixture of these solvents.

The volatile oil(s), in one embodiment, is present in an amount ranging from 0% to 95.5% relative to the total weight of the composition, such as from 2% to 75% or, for example, from 10% to 45%. This amount will be adapted by a person skilled in the art according to the desired staying power or long wearing properties.

The compositions of the present invention may also contain one or more sun screening agents.

Sunscreens according to this invention which are chemical absorbers actually absorb harmful ultraviolet radiation. It is well known that chemical absorbers are classified, depending on the type of radiation they protect against, as either UV-A or UV-B absorbers. UV-A absorbers generally absorb radiation in the 320 to 400 nm region of the ultraviolet spectrum. UV-A absorbers include anthranilates, benzophenones, and dibenzoyl methanes. UV-B absorbers generally absorb radiation in the 280 to 320 nm region of the ultraviolet spectrum. UV-B absorbers include *p*-aminobenzoic acid derivatives, camphor derivatives, cinnamates, and salicylates.

Classifying the chemical absorbers generally as UV-A or UV-B absorbers is accepted within the industry. However, a more precise classification is one based upon the chemical properties of the sunscreens. There are eight major classifications of sunscreen chemical properties which are discussed at length in "Sunscreens - Development, Evaluation and Regulatory Aspects," by N. Shaath et al., 2nd. Edition, pages 269-273, Marcel Dekker, Inc. (1997). This discussion, in its entirety, is incorporated by reference herein.

The sunscreens which may be formulated according to the present invention typically comprise chemical absorbers, but may also comprise physical blockers. Exemplary sunscreens which may be formulated into the compositions of the present invention are chemical absorbers such as *p*-aminobenzoic acid derivatives, anthranilates, benzophenones, camphor derivatives, cinnamic derivatives, dibenzoyl methanes (such as avobenzone also known as Parsol®1789), diphenylacrylate derivatives, salicylic derivatives, triazine derivatives, benzimidazole compounds, bis-benzoazolyl derivatives, methylene bis-(hydroxyphenylbenzotriazole) compounds, the sunscreen polymers and silicones, or mixtures thereof. These are variously described in U.S. Patents Nos. 2,463,264, 4,367,390, 5,166,355 and 5,237,071 and in EP-0,863,145, EP-0,517,104, EP-0,570,838, EP-0,796,851, EP-0,775,698, EP-0,878,469, EP-0,933,376, EP-0,893,119, EP-0,669,323, GB-2,303,549, DE-1,972,184 and WO-93/04665, also expressly incorporated by reference. Also exemplary of the sunscreens which may be formulated into the compositions of this invention are physical blockers such as cerium oxides, chromium oxides, cobalt oxides, iron oxides, red petrolatum, silicone-treated titanium dioxide, titanium dioxide, zinc oxide, and/or zirconium oxide, or mixtures thereof.

A wide variety of sunscreens is described in U.S. Patent No. 5,087,445, issued to Haffey et al. on February 11, 1992; U.S. Patent No. 5,073,372, issued to Turner et al. on December 17, 1991; and Chapter VIII of Cosmetics and Science and Technology by Segarin et al., pages 189 et seq. (1957), all of which are incorporated herein by reference in their entirety.

Sunscreens which may be formulated into the compositions of the instant invention are those selected from among: aminobenzoic acid, amyldimethyl PABA, cinoxate, diethanolamine *p*-methoxycinnamate, digalloyl trioleate, dioxybenzone, 2-ethoxyethyl *p-*methoxycinnamate, ethyl 4-bis(hydroxypropyl)aminobenzoate, 2-ethylhexyl-2-cyano-3,3-diphenylacrylate, ethylhexyl *p*-methoxycinnamate, 2-ethylhexyl salicylate, glyceryl aminobenzoate, homomenthyl salicylate, homosalate, 3-imidazol-4-ylacrylic acid and ethyl ester, methyl anthranilate, octyldimethyl PABA, 2-phenylbenzimidazole-5-sulfonic acid and salts, red petrolatum, sulisobenzone, titanium dioxide, triethanolamine salicylate, *N, N, N-*trimethyl-4-(2-oxoborn-3-ylidene methyl)anillinium methyl sulfate, and mixtures thereof.

Sunscreens active in the UV-A and/or UV-B range can also include: p-aminobenzoic acid, oxyethylene (25 mol) p-aminobenzoate,2-ethylhexyl p-dimethylaminobenzoate,ethyl N-oxypropylene p-aminobenzoate, glycerol p-aminobenzoate, 4-isopropylbenzyl salicylate, 2-ethylhexyl 4-methoxycinnamate, methyl diisopropylcinnamate,isoamyl 4-methoxycinnamate, diethanolamine 4-methoxycinnamate,3-(4'-trimethylammunium)-benzyliden-boman-2-one methylsulfate, 2-hydroxy-4-methoxybenzophenone, 2-hydroxy-4-methoxybenzophenone-5-sulfonate, 2,4-dihydroxybenzophenone, 2,2',4,4'-tetrahydroxybenzophenone, 2,2'-dihydroxy-4,4'dimethoxybenzophenone, 2-hydroxy-4-n-octoxybenzophenone, 2-hydroxy-4-methoxy-4'-methoxybenzophenone, -(2-oxoborn-3-ylidene)-tolyl-4-sulfonic acid and soluble salts thereof, 3-(4'-sulfo)benzyliden-bornan-2-one and soluble salts thereof, 3-(4'methylbenzylidene)-d,1-camphor, 3-benzylidene-d,1-camphor, benzene 1,4-di(3-methylidene-10-camphosulfonic) acid and salts thereof (the product Mexoryl SX described in U.S. Patent No. 4,585,597 issued to Lange et al. on April 29, 1986),urocanic acid, 2,4,6-tris[p-(2'-ethylhexyl-1'-oxycarbonyl)-anilino]-1,3,5-triazine, 2-[(p-(tertiobutylamido)anilino]-4,6-bis-[(p-(2'-ethylhexyl-1'-oxycarbonyl)anilino]-1,3,5-triazine, 2,4-bis {[4-(2-ethyl-hexyloxy)]-2-hydroxy]-phenyl}-6-(4-methoxy-phenyl)-1,3,5-triazine ("TINOSORB S" marketed by Ciba), the polymer of N-(2 et 4)-[(2-oxoborn-3-yliden)methyl]benzyl]-acrylamide, 1,4-bisbenzimidazolyl-phenylen-3,3',5,5'-tetrasulfonic acid and salts thereof, the benzalmalonate-substituted polyorganosiloxanes, the benzotriazole- substituted polyorganosiloxanes (Drometrizole Trisiloxane), dispersed 2,2'-methylene-bis-[6-(2H-benzotriazol-2-yl)-4-(1,1,3,3-tetramethylbutyl)phenol] such as that marketed under the trademark MIXXIM BB/100 by Fairmount Chemical, or micronized in dispersed form thereof such as that marketed under the trademark TINOSORB M by Ciba-Geigy, and solubilized 2,2'-methylene-bis-[6-(2H-benzotriazol-2-yl)-4-(methyl)phenol] such as that marketed under the trademark MIXXIM BB/200 by Fairmount Chemical. Typically combinations of one of more of these sunscreens are used.

The dibenzoyl methane derivatives other than avobenzone are described, for example, in FR-2,326,405, FR-2,440,933 and EP-0,114,607, hereby expressly incorporated by reference.

Other dibenzoyl methane sunscreens other than avobenzone include (whether singly or in any combination): 2-methyldibenzoylmethane; 4-methyldibenzoylmethane; 4-isopropyldibenzoylmethane; 4-tert.-butyldibenzoylmethane; 2,4-dimethyldibenzoylmethane; 2,5-dimethyldibenzoylmethane; 4,4'-diisopropyldibenzoylmethane; 4,4'-dimethoxydibenzoylmethane; 2-methyl-5-isopropyl-4'-methoxydibenzoylmethane; 2-methyl-5-tert.-butyl-4'-methoxydibenzoylmethane; 2,4-dimethyl-4'-methoxydibenzoylmethane; 2,6-dimethyl-4-tert.-butyl-4'-methoxydibenzoylmethane; Additional sunscreens that can be used are described in pages 2954-2955 of the *International Cosmetic Ingredient Dictionary and Handbook* (9^{th} ed. 2002).

The compositions of the invention may further comprise at least one additional fatty material. The at least one additional fatty material may, for example, be chosen from gums, fatty materials pasty at ambient temperature, and resins.

The compositions of the invention may further include formulation aids which are usually employed in the field of application envisaged. The formulation aids used in the present invention can be, but are not limited to, fatty substances. Useful fatty substances include, but are not limited to, organic and organosilicone emulsifiers for water-in-oil systems. Examples of organic emulsifiers include any ethoxylated surfactants known in the art such as Polysorbate-20, Laureth-7, Laureth-4, Sepigel® 305 available from SEPPIC and other similar ingredients disclosed in the *International Cosmetic Ingredient Dictionary and Handbook Vol. 4* (9^{th} ed. 2002), more particularly the emulsifiers disclosed on pages 2962-2971. The disclosure of the *International Cosmetic Ingredient Dictionary and Handbook Vol. 4*, pages 2962-2971, is hereby incorporated by reference. Examples of organosilicone emulsifiers include cetyl dimethicone copolyol-polyglyceryl-4-isostearate-hexylaurate (ABIL® WE 09) available from Goldschmidt Chemical Corporation, Cetyl Dimethicone Copolyol (ABIL® EM 90), (ABIL® EM 97), Laurylmethicone Copolyol (5200), Cyclomethicone (and) Dimethicone Copolyol (DC 5225 C and DC 3225 C) available from GE Silicones, Cyclopentasiloxane & Dimethicone Copolyol (GE SF 1528) or any other formulation aids known by one of skill in the art. Other fatty substances useful as formulation aids include but are not limited to, silicones in esterified or unesterified liquid form or in esterified solid form, such as behenate dimethicone; and non-silicone fatty substances including oils such as vegetable and mineral oil; animal and/or synthetic waxes such as beeswax, paraffin, rice bran wax, candelilla wax, carnauba wax and derivatives thereof; and hydrocarbon gels or bentone type gels, such as Gel SS71, Gel EA2786, Quaternium-18 Bentonite, 38 CE, Gel ISD V or Gel ISD. In one embodiment, the wax is a synthetic beeswax, such as the beeswax sold as Kester K82P (Whittaker, Clark, and Daniels, Inc.) which is made of a range of hydroxyl polyesters with an average chain length of 66 carbon atoms, C20-40 alkyl hydroxystearyl stearate (CAS 151661-95-9).

These substances may be selected variously by the person skilled in the art in order to prepare a composition which has the desired properties, for example, consistency or texture.

Plasticizers may also be added to the compositions to improve the flexibility and cosmetic properties of the resulting formulation. Plasticizers are materials which soften synthetic polymers. They are frequently required to avoid brittleness and cracking of film formers. One skilled in the art may routinely vary the amount of plasticizer desired based on the properties desired and the application envisaged. Plasticizers useful in the practice of the invention include lecithin, polysorbates, dimethicone copolyol, glycols, citrate esters, a polyol, dimethicone, and other similar ingredients disclosed in the *International Cosmetic Ingredient Dictionary and Handbook Vol. 4* (9^{th} ed. 2002), more particularly the plasticizers disclosed on page 2927. The disclosure of the *International Cosmetic Ingredient Dictionary and Handbook Vol. 4*, page 2927, is hereby incorporated by reference.

The composition of the present invention may also further comprise at least one suitable additive commonly used in the field concerned chosen from coloring agents, antioxidants, essential oils, preserving agents, fragrances, fillers, pasty fatty substances, waxy fatty substances, neutralizing agents, liposoluble polymers, and cosmetically active agents and dermatological active agents such as, for example, emollients, moisturizers, vitamins and essential fatty acids. The compositions of the invention may also be optionally thickened with an aqueous-phase thickener or gelled with a gelling agent and/or containing ingredients soluble in water.

The compositions may also contain one or more additional waxes, synthetic or natural. As used herein, a "wax" may be any lipophilic fatty compound. Non-limiting examples of such waxes include waxes of natural origin, such as beeswax, carnauba wax, candelilla wax, ouricury wax, Japan wax, cork fiber wax, sugar cane wax, paraffin waxes, lignite wax, microcrystalline waxes, lanolin wax, montan wax and ozokerites, hydrogenated oils such as hydrogenated jojoba oil, jojoba esters, waxes of synthetic origin, such as polyethylene waxes derived from polymerization of ethylene, waxes obtained by Fischer-Tropsch synthesis, fatty acid esters and glycerides, and silicone waxes such as derivatives of poly(di)methylsiloxane.

Another embodiment of the invention relates to a skin lip, or keratinous material care or make-up composition comprising the components of the composition as described herein.

Another embodiment of the invention relates to increasing the hydration properties of at least one polyol in a composition by adding poly(2-hydroxymethyl methacrylate) to a composition comprising at least one polyol.

Additionally, an embodiment of the invention relates to a method of providing a moisturizing benefit to the skin, lips, or keratinous materials by applying to such skin, lips, and/or keratinous material in need thereof the compositions as described herein such that the skin, lips, and/or keratinous material become moisturized.

In the method of providing a moisturizing benefit, the composition can be applied to dry skin, lips and/or keratinous material as a treatment, the composition can be applied prior to any visible detection of dryness occurs as a preventative, or the composition can be incorporated into an individual's routine hygiene and healthcare maintenance regimen.

The present invention also envisages kits and/or prepackaged materials suitable for consumer use containing one or more compositions according to the description herein to provide a moisturizing benefit to the skin, lips, and/or keratinous material. The packaging and application device for any subject of the invention may be chosen and manufactured by persons skilled in the art on the basis of their general knowledge, and adapted according to the nature of the composition to be packaged. Indeed, the type of device to be used can be in particular linked to the consistency of the composition, in particular to its viscosity; it can also depend on the nature of the constituents present in the composition, such as the presence of volatile compounds.

In another embodiment, the present invention is directed to a process of making a moisturizing composition with at least one polyol and the poly(2-hydroxyethyl methacrylate), the process including a method of mixing the two ingredients directly or first mixing separately with additional compositional components described herein and then mixing the ingredients together.

Examples

The following lipstick formulations were prepared. Numbers are percent by weight of the composition.

| Seq | Ingredient | Ex. 1 | Ex. 2 | Ex. 3 | Ex. 4 | Ex. 5 |
|---|---|---|---|---|---|---|
| | polyglyceryl-2 diisostearate | 8.00 | 7.49 | 7.49 | 8.00 | 8.00 |
| | glycerin | | 3.00 | 3.00 | | |
| | BHT | 0.03 | 0.03 | 0.03 | 0.03 | 0.03 |
| | hydrogenated polyisobutene | 5.85 | 5.47 | 5.47 | 5.85 | 5.85 |
| | Jojoba oil | 4.70 | 4.40 | 4.40 | 4.70 | 4.70 |
| A | Butyrospermum Parkii (Shea Butter) Fruit | 3.00 | 2.82 | 2.82 | 3.00 | 3.00 |
| | PVP/hexadecene copolymer | 2.00 | 1.87 | 1.87 | 2.00 | 2.00 |
| | diisopropyl dimer dilinoleate | 17.48 | 17.01 | 17.01 | 17.48 | 17.41 |
| | bis-diglyceryl polyacyladipate-2 | 5.00 | 4.68 | 4.68 | 5.00 | 5.00 |
| | squalane (vegetable) | 4.70 | 4.40 | 4.40 | 4.70 | 4.70 |
| | isopropylparaben, isobutylparaben | 0.40 | 0.37 | 0.37 | 0.40 | 0.40 |
| | hydrogenated polyisobutene | 6.00 | 5.62 | 5.62 | 6.00 | 6.00 |
| | polyethylene | 6.05 | 6.05 | 6.05 | 6.05 | 6.05 |
| B | ozokerite | 4.40 | 4.40 | 4.40 | 4.40 | 4.40 |
| | microcrystalline wax | 3.00 | 3.00 | 3.00 | 3.00 | 3.00 |
| | octyldodecanol | 5.69 | 5.69 | 5.69 | 5.69 | 5.69 |
| | Allantoin | 0.60 | 0.60 | 0.60 | 0.60 | 0.60 |
| C | disteardimonium hectorite | 0.50 | 0.50 | 0.50 | 0.50 | 0.50 |
| | propylene carbonate | 0.05 | 0.05 | 0.05 | 0.05 | 0.05 |
| | Pigments | 6.96 | 6.96 | 6.96 | 6.96 | 5.38 |
| D | Pearl and filler | 1.50 | 3.50 | 1.50 | 3.50 | 5.16 |
| | 2-hydroxyethyl methacrylate | 2.00 | | 2.00 | | |
| | Proliposome moisturizer | | | | | 2.00 |
| | BHT | 0.02 | 0.02 | 0.02 | 0.02 | 0.02 |
| | tocopheryl acetate | 1.00 | 1.00 | 1.00 | 1.00 | 1.00 |
| | ethylhexyl methoxycinnamate | 7.50 | 7.50 | 7.50 | 7.50 | 7.50 |
| | Ascorbyl palmitate | 0.02 | 0.02 | 0.02 | 0.02 | 0.02 |
| | Panthenyl triacetate | 0.02 | 0.02 | 0.02 | 0.02 | 0.02 |
| E | octyldodecanol, tocopheryl acetate, silica | 1.00 | 1.00 | 1.00 | 1.00 | 1.00 |
| | octyldedecyl neopentanoate | 1.00 | 1.00 | 1.00 | 1.00 | 1.00 |
| | retinyl palmitate | 0.03 | 0.03 | 0.03 | 0.03 | 0.03 |
| | cholesteryl/behenyl/octyldodecyl lauroyl glutamate | 1.50 | 1.50 | 1.50 | 1.50 | 1.50 |
| | Total= | 100.00 | 100.00 | 100.00 | 100.00 | 100.00 |

| Seq | Ingredient | Ex. 6 |
|---|---|---|
| | polyglyceryl-2 diisostearate | 10.00 |
| | glycerin | 3.00 |
| | BHT | 0.03 |
| | hydrogenated polyisobutene | 5.85 |
| | Jojoba oil | 4.70 |
| A | Butyrospermum Parkii (Shea Butter) Fruit | 3.00 |
| | PVP/hexadecene copolymer | 5.00 |
| | diisopropyl dimer dilinoleate | 17.48 |
| | bis-diglyceryl polyacyladipate-2 | 5.00 |
| | squalane (vegetable) | 4.70 |
| | isopropylparaben, isobutylparaben | 0.40 |
| | octyldodecanol | 5.69 |
| B | Allantoin | 0.60 |
| | disteardimonium hectorite | 0.50 |
| | propylene carbonate | 0.05 |
| | polyethylene | 6.05 |
| C | ozokerite | 4.40 |
| | hydroxy polyester | 2.00 |
| | pigment | 6.96 |
| D | mica | 2.50 |
| | 2-hydroxyethyl methacrylate | 2.00 |
| | | |
| | BHT | 0.02 |
| | tocopheryl acetate | 1.00 |
| | ethylhexyl methoxycinnamate | 7.50 |
| | Ascorbyl palmitate | 0.02 |
| E | Panthenyl triacetate | 0.02 |
| | retinyl palmitate | 0.03 |
| | cholesteryl/behenyl/octyldodecyl lauroyl glutamate | 1.50 |
| | | |
| | Total= | 100.00 |

| Seq | Ingredient | Ex. 7 |
|---|---|---|
| | POLYGLYCERYL-3 DIISOSTEARATE | 9.00 |
| | glycerin | 3.00 |
| | BHT | 0.05 |
| | hydrogenated polyisobutene | 12.90 |
| | glyceryl oleate | 11.00 |
| | octyldodecanol | 5.00 |
| A | ethylhexyl methoxycinnamate | 7.50 |
| | Butyrospermum Parkii (Shea Butter) Fruit | 2.00 |
| | PVP/hexadecene copolymer | 5.00 |
| | diisopropyl dimer dilinoleate | 12.00 |
| | bis-diglyceryl polyacyladipate-2 | 5.00 |
| | isopropylparaben, isobutylparaben | 0.40 |
| | polyethylene | 6.40 |
| B | ozokerite | 3.00 |
| | Kester Wax K 82 P | 1.75 |
| | Pigments | 8.40 |
| | 2-hydroxyethyl methacrylate | 1.80 |
| C | | |
| | Allantoin/DID, 60/40 | 1.00 |
| | | |
| | tocopheryl acetate | 0.50 |
| | avocado oil | 2.00 |
| | squalane (vegetable) | 2.00 |
| D | Ascorbyl palmitate | 0.01 |
| | Panthenyl triacetate | 0.01 |
| | retinyl palmitate | 0.01 |
| | | |
| | Total= | 100.00 |

The components of each phase can be mixed separately and then mixed together using the appropriate mixers. The resultant composition can then be formed into a stick

Moisturizing efficacy and cosmetic attribute testing

The formulation of Example 4 was compared to Example 4 with 3% glycerin, Example 4 with 3% glycerin and 2% polyHEMA powder, and Example 4 with 2% proliposome moisturizer. Each of the formulations were assessed by human panelists under the criteria of overall appearance at 2 hours, ease of application, coverage, gloss/shine, moisturizing feeling, heavy feeling, overall appearance, 2 hour wear off, moisturizing at 2 hours, feathering, bleeding, comfort, sticky/tacky feel, and amount of gloss. The formulation with 3% glycerin and 2% polyHEMA powder was found to be the most moisturizing, comfortable composition with the least amount of wear off after 2 hours and preferred overall by the panelists.

Hydration test

The formulation of Example 4 was compared to Example 4 with 2% polyHEMA powder, Example 4 with 3% glycerin, Example 4 with 3% glycerin and 2% polyHEMA powder in a hydration test. The samples were applied to the forearm of the panelists (27) and hydration values were measured by a Novameter at 1 hour, 2 hours and 3 hours. The formulation with 3% glycerin and 2% polyHEMA was found to be the most hydrated formula at 2 hours and 3 hours.

Sensory test

The formulations of Example 4, Example 4 with 3% glycerin, Example 4 with 3% glycerin and 2% polyHEMA were compared with respect to feathering at 2 and 4 hours. Moisturizing and comfort were also assessed.

The formulation of Example 4 with 3% glycerin and 2% polyHEMA exhibited the least amount of feathering and bleeding. At 4 hours wear, the Example 4 formulation with 3% glycerin and 2% polyHEMA remained more moisturizing, retained more coverage, had more shine and was more comfortable than the other two formulas.

Obviously, numerous modifications and variations of the present invention are possible in light of the above teachings. It is therefore to be understood that within the scope of the appended claims, the invention may be practiced otherwise than as specifically described herein.

## Claims

1. Composition suitable for application to keratinous material, comprising moisturizing effective amounts of at least one polyol and poly(2-hydroxyethyl methacrylate).

2. Composition according to claim 1, wherein the at least one polyol is present in an amount from 0.5 to 75 % by weight of the composition.

3. Composition according to claim 2, wherein the at least one polyol is present in an amount from 1 to 5 % by weight of the composition.

4. Composition according to anyone of claims 1 to 3, wherein the poly(2-hydroxyethyl methacrylate) is present in an amount from 0.1 to 50 % by weight of the composition.

5. Composition according to claim 4, wherein the poly(2-hydroxyethyl methacrylate) is present in an amount from 1 to 5% by weight of the composition.

6. Composition according to anyone of claims 1 to 5, wherein the polyol is selected from polyhydric alcohols, ethoxylated and propoxylated polyols, polysaccharides, and mixtures thereof.

7. Composition according to claim 6, wherein the polyol is selected from glycerin, panthenol, hexylene glycol, propylene glycol, butylene glycol, alkanediols, polyethylene glycol, polypropylene glycol, sorbitol, and mixtures thereof.

8. Composition according to anyone of claims 1 to 7, wherein the at least one polyol is glycerin.

9. Composition according to claim 8, wherein the glycerin is present in an amount from 0.5 to 75% by weight of the composition.

10. Composition according to claim 9, wherein the glycerin is present in an amount from 1 to 5% by weight of the composition.

11. Composition according to anyone of claims 1 to 10, further comprising one or more sunscreen agents.

12. The composition according to anyone of claims 1 to 11, further comprising one or more film-forming polymers.

13. Composition according to anyone of claims 1 to 12, further comprising one or more pigments.

14. Composition according to anyone of claims 1 to 13, wherein the composition is anhydrous.

15. Composition according to anyone of claims 1 to 14, which is in the form of a cream, gel, lotion, or stick.

16. Composition according to anyone of claims 1 to 15, which is a lipstick.

17. Cosmetic method of providing a moisturizing benefit to one or more keratinous materials, comprising applying a composition comprising at least one poly(2-hydroxyethyl methacrylate) and at least one polyol to a keratinous material in need thereof, in an amount effective to provide a moisturizing benefit thereto.

18. Method according to claim 17, wherein the composition is according to anyone of claims 2 to 16.

19. Method of manufacturing a composition according to anyone of claims 1 to 16, comprising mixing the at least one polyol and the poly(2-hydroxyethyl methacrylate).
